# EUROPÄISCHE PATENTSCHRIFT

(11) **EP 1 339 397 B1**
(45) Veröffentlichungstag und Bekanntmachung des Hinweises auf die Patenterteilung: **11.08.2004**
(21) Anmeldenummer: 01990513.2
(22) Anmeldetag: 05.12.2001
(51) Int. Cl.: A61K 9/70

(54) **ABSORPTIONSMITTEL UND KANALBILDNER ENTHALTENDE WIRKSTOFFUNDURCHLÄSSIGE DECKSCHICHT ODER ABZIEHBARE SCHUTZSCHICHT EINES TRANSDERMALEN THERAPEUTISCHEN SYSTEMS**
ABSORBING AGENTS AND COVER LAYER WHICH IS IMPERMEABLE TO ACTIVE SUBSTANCES AND WHICH CONTAINS CHANNEL-FORMERS OR REMOVABLE PROTECTIVE LAYER OF A TRANSDERMAL THERAPEUTIC SYSTEM
COUCHE DE RECOUVREMENT OU COUCHE PROTECTRICE PELABLE COMPORTANT DES ABSORBANTS ET DES AGENTS DE FORMATION DE CANAUX, POUR UN SYSTEME THERAPEUTIQUE TRANSDERMIQUE

(30) Priorität: 06.12.2000 DE 10060852
(43) Veröffentlichungstag der Anmeldung: 03.09.2003
(73) Patentinhaber: HEXAL AG, 83607 Holzkirchen (DE)
(72) Erfinder: BEIER, Cornelia, 83607 Holzkirchen (DE); KIBELE, Ralf, 83607 Holzkirchen (DE)
(74) Vertreter: Boeters, Hans Dietrich, Dr.
(86) Internationale Anmeldenummer: PCT/EP2001/014280
(87) Internationale Veröffentlichungsnummer: WO 2002/045700

(56) Entgegenhaltungen:
- EP-A- 0 399 765
- WO-A-97/32663
- WO-A-98/39231
- WO-A-99/61855

## Beschreibung

Die Erfindung betrifft eine wirkstoffundurchlässige Deckschicht oder eine abziehbare Schutzschicht eines transdermalen therapeutischen Systems, die Absorptionsmittel und Kanalbildner enthält.

Die in herkömmlichen transdermalen therapeutischen Systemen verwendeten Deckschichten sind Folien aus Acetal, Acrylat, Acrylonitril-Butadien-Styrol, Acrylonitril-(methylmethacrylat) -Copolymer, Acrylonitril-Copolymer, Ethylenethylacrylat, Ethylenmethylacrylat, Ethylenvinylacetat, Ethylenvinylacetat-Copolymer, Ethylenvinylalkohol-Polymer, Ionomere, Nylon (Polyamid), Nylon-Copolymer, Polybutylen, Polycarbonat, Polyester, Polyethylenterephthalat, thermoplastisches PolyesterCopolymer, Polyethylen-Copolymer (high density), Polyethylen (high-molecular-weight, high-density), Polyethylen (intermediate-molecular-weight, high-density), Polyethylen (linear low density), Polyethylen (low density), Polyethylen (medium density), Polyethylenoxid, Polyimid, Polypropylen, Polypropylen (coated), Polypropylen (oriented), Polystyrol, Polyurethan, Polyvinylacetat, Polyvinylchlorid, Polyvinylidenchlorid und/oder Styrol- Acrylonitril, die bei Bedarf metallisiert oder pigmentiert sein können. Sie haben die Aufgabe, das wirkstoffhaltige System zu stabilisieren und vor Außeneinflüsssen zu schützen. Die Deckschicht ist wirkstoffundurchlässig und verhindert somit eine Diffusion des Wirkstoffes nach außen.

Die in transdermalen therapeutischen Systemen verwendeten abziehbaren Schutzschichten sind ebenfalls wirkstoffundurchlässig und bestehen üblicherweise aus Polyester, Polyethylen, Polypropylen, Polysiloxan, Polyacrylat, Ethylenvinylacetat, Polyurethan, Polyisobuten oder Papier, meistens mit Silikon- und/oder Polyethylen beschichtet, oder ein Verbund aus diesen. Sie haben ebenfalls die Aufgabe, das wirkstoffhaltige System zu stabilisieren und vor Außeneinflüsssen zu schützen. Die abziehbare Schutzschicht ist wirkstoffundurchlässig und verhindert somit eine Diffusion des Wirkstoffes während der Lagerung nach außen.

Transdermale therapeutische Systeme werden üblicherweise in Sachets verpackt. Die Sachets bestehen aus in der Regel mit Aluminium beschichtetem Verbundmaterial. Durch diese Beschichtung wird die Feuchtigkeits- und Sauerstoffdurchlässigkeit der Verpackung gesenkt. Die Durchlässigkeit ist abhängig von der eschichtungsdicke. Je dicker die Aluminiumbeschichtung, desto umweltbelastender und kostenintensiver ist die Herstellung und Entsorgung der Verpackung. Eine vollständige Undurchlässigkeit kann aber praktisch nicht erreicht werden. Aus diesem Grunde ist das transdermale therapeutische System immer einem gewissen Feuchtigkeitsgrad ausgesetzt. Zudem gibt das transdermale therapeutischen Systemen nach der Herstellung eine gewisse Restfeuchtigkeit an die Umwelt ab. Um eine angemessene Lagerstabilität zu gewährleisten, kann in das Sachet ein Inlay eingebracht werden, das ein Trocknungsmittel enthält. Die Verpackung des transdermalen therapeutischen Systems wird dadurch aufwendiger und wesentlich teurer. Geruchsintensive Stoffe, wie Amine und Polymermonomere, die im Laufe der Lagerung aus dem Wirkstoff oder der Kleberschicht freigesetzt werden, können mit diesem Verfahren nicht oder nur partiell entfernt werden.

Die Aufgabe der Erfindung ist es, die Lagerstabilität von transdermalen therapeutischen Systemen zu erhöhen, indem der negative Einfluß von Feuchtigkeit, Sauerstoff, freien Aminen und/oder freien Polymermonomeren auf die Stabilität der transdermalen therapeutischen Systeme auf ein Minimum reduziert wird. Die Herstellung der transdermalen therapeutischen Systeme soll dabei auf die übliche Art und Weise, ohne zusätzliche aufwendige Produktionsschritte, erfolgen, wobei die Kosten für die Verpackung im gleichen Zuge gesenkt werden sollen.

Die erfindungsgemäße Aufgabe konnte nun überraschenderweise gelöst werden, indem als wirkstoffundurchlässige Deckschicht oder abziehbare Schutzschicht des transdermalen therapeutischen Systems ein Polymer in Form einer Folie verwendet wird, die entweder die Absorptionsmittel und Kanalbildner direkt beinhaltet oder die mit einem diese Stoffe beinhaltendem Polymerträger beschichtet ist. Die Beschichtung kann entweder vollflächig über die ganze Folie oder in Mustern (z.B. Gitterform) direkt bei der Produktion aufgebracht werden.

Die Firma Capitol Specialty Plastics hat mehrere Patentanmeldungen (WO 00/17259, WO 00/17260, WO 00/16884, WO 99/62697, WO 99/61856, WO 98/39231, WO 99/61855, WO 00/17258, WO 99/63288, US 5,911,937), die Polymere beanspruchen, welche absorbierende Stoffe, freisetzende Stoffe, Kanalbildner, etc. beinhalten. Diese Polymere werden hauptsächlich zur Entfernung von Feuchtigkeit in Verpackungen verwendet. Dabei liegen sie entweder als Inlays vor, die als Einschub in die Verpackungen eingebracht werden, oder die Verpackungen werden damit ausgekleidet. Die Dicke der beschriebenen Inlays und Beschichtungsfolien beträgt ≥ 400 µm. Das hat den Nachteil, daß diese Inlays oder Beschichtungen einen zusätzlichen Produktionsschritt erfordern und einen weiteren Kostenfaktor darstellen.

Durch die erfindungsgemäße Deckschicht bzw. abziehbare Schützschicht kann die Dicke der Aluminiumbeschichtung der Verpackung stark reduziert werden. Das entlastet die Umwelt und verbilligt die Verpackung. Der Herstellungsprozeß muß nicht umgestellt werden. Die nach dem Stand der Technik übliche Herstellung kann weiterhin durchgeführt werden. Außerdem muß kein zusätzliches Trockenelement in die Verpackung eingefügt werden. Die Herstellungskosten des TTS können somit wesentlich verringert werden.

Das transdermale therapeutische System (TTS) kann über lange Zeit stabil gelagert werden. Auch unter extremen Bedingungen, wie z.B. in den Tropen, kann das transdermale therapeutische System ohne zusätzlichen Elemente über lange Zeit ohne Stabilitätsverlust gelagert werden. Ebenso können TTS mit feuchtigkeitslabilen Wirkstoffen ohne Stabilitätsprobleme aufbewahrt werden. Es kann in diesen Fällen sogar die Lagerdauer verlängert werden.

Die erfindungsgemäße wirkstoffundurchlässige Deckschicht und/oder abziehbare Schutzschicht eines transdermalen therapeutischen Systems besteht aus einem Polymeren, (i) das entweder die Absorptionsmittel und Kanalbildner direkt beinhaltet oder (ii) das mit einem diese Stoffe beinhaltendem Polymerträger beschichtet ist. Der Polymerträger kann entweder vollflächig über die ganze Folie oder in Mustern direkt bei der Produktion aufgebracht werden. Ist ein eigener Polymerträger vorgesehen, so können Deckschicht und/oder Schutzschicht und der Polymerträger entweder aus gleichen oder verschiedenen Materialien bestehen.

Die für die Mischung aus Polymer (für Polymerträger oder Deckschicht und/oder abziehbare Schutzschicht), Absorptionsmittel und Kanalbildner verwendeten Polymere können Thermoplaste sein; sie können z.B. Polyolefine, wie Polyethylen und/oder Polypropylen, Polyisoprene, Polybutadiene, Polybutene, Polysiloxane, Polyamide, Ethylen-Vinylacetat-Copolymere, Ethylen-Methacrylat-Copolymere, Polystyrole, Polyester, Polyanhydride, Polyacrylatnitrile, Polysulfonate, Polyesteramide, Polyacrylatester, Propylen-Maleinsäureanhydrid, Polyethylen-Maleinsäureanhydrid, Polyethylen-Urethane, Polyethylen-Ethylvinylalkohole, Polyethylen-Nylon und/oder Polyurethane sein.

Weiterhin kann es sich bei dem Polymer um ein vernetzbares Polymer handeln, das thermisch oder strahlungsvernetzbar, insbesondere UV-vernetzbar ist. Es kann also das Polymer des Polymerträgers oder der Deckschicht und/oder der abziehbaren Schutzschicht vernetzbar sein. Ist ein eigener Polymerträger vorgesehen, so kann ein thermisch vernetzbares Polymer kalt oder in der Wärme auf die Deckschicht und/oder Schutzschicht aufgetragen werden, während ein strahlungsvernetzbares Polymeres kalt oder heiß aufgetragen werden kann. Die Vernetzung kann nach dem jeweiligen Auftrag erfolgen.

Der Gehalt an Polymer beträgt 10 - 90 Gew. % bezogen auf das Gesamtgewicht der Mischung aus Polymer, Kanalbildner und Absorptionsstoffen, unabhängig davon ob es direkt als Folie oder als Beschichtung verwendet wird.

Die in der vorliegenden Erfindung verwendeten Kanalbildner können hydrophile Stoffe wie z.B. Polyglykole, Ethylvinylalkohole, Glycerin, Pentaerithritol, Polyvinylalkohole, Polyvinylpyrrolidon, Vinylpyrrolidon, N-Methylpyrrolidon, Polysaccharide, Saccharide und/oder Zuckeralkohole sein. Als Polyglykole werden Polyethylenglykol und/oder Polypropylenglykol bevorzugt. Als Saccharide können z.B. Glucose, Mannose, Galactose und/oder Fructose verwendet werden. Als Zuckeralkohole kommen z.B. Mannitol, Sorbitol, Hexitol, Dulcitol, Xylitol, Ribitol und/oder Erythrol in Frage. Unter Polysaccharide können z.B. Dextrine und/oder hydrolisierte Stärke verstanden werden.

Der Gehalt an Kanalbildnern kann 10-40 Gew. %, bezogen auf das Gesamtgewicht der Mischung aus Polymer, Kanalbildner und Absorptionsstoffen, betragen.

Es können verschieden Arten von Absorptionsmitteln in die Deckschicht oder abziehbare Schutzschicht eingebracht werden.

Eine erfindungsgemäße Ausführungsform beinhaltet Trockenmittel als Absorptionsmittel. Es gibt drei verschiedene Gruppen von Trockenmitteln.

Eine Gruppe beinhaltet chemische Stoffe, die mit Wasser Hydrate bilden. Beispiele derartiger chemischer Stoffe können wasserfreie Salze sein, die dazu neigen, Wasser oder Feuchtigkeit zu absorbieren, und dabei ein stabiles Hydrat bilden. Bei dieser Reaktion wird die Feuchtigkeit gebunden und deren Freisetzung durch eine chemische Reaktion verhindert.

Die zweite Gruppe der Trockenmittel enthält Stoffe, die reaktiv sind. Diese Stoffe reagieren mit Wasser oder Feuchtigkeit, indem sie einen neuen Stoff bilden. Die neu gebildeten Stoffe sind normalerweise bei niedrigen Temperaturen stabil. Nur unter Aufwendung von hoher Energie ist deren Bildung wieder reversibel. Diese Art von Trockenmitteln wird hauptsächlich zum Trocknen von Lösungsmitteln und als wasserabsorbierendes Material bei Polymeren, die selber in einem feuchtigkeitsreduzierten Zustand bleiben müssen, verwendet.

Die dritte Gruppe der Trockenmittel bindet die Feuchtigkeit durch physikalische Absorption. Die Trockenmittelteilchen haben eine feine Kapillarstruktur, so daß die Feuchtigkeit in diese Kapillare gezogen wird. Die Porengröße der Kapillare sowie die Kapillardichte bestimmen die Absorptionseigenschaften des Trockenmittels. Beispiele dieser Art der Trockenmittel sind Molekularsiebe, Siliziumgele, Erden (z.B. Montmorillimiterde), bestimmte synthetische Polymere (z.B. Polymere, die in Babywindeln verwendet werden) und Stärken.
Diese Gruppe der Trockenmittel wird aufgrund ihrer Inertheit und Wasserunlöslichkeit bevorzugt.

Eine bevorzugte erfindungsgemäße Ausführungsform beinhaltet als Trockenmittel Molekularsiebe mit einer Porengröße von 3 - 15 Angström.

Eine weitere erfindungsgemäße Ausführungsform beinhaltet Siliziumgel mit einer Porengröße von 24 Angström.

Als weitere mögliche Absorptionsmittel können Metalle und Legierungen, wie z.B. Nickel, Kupfer, Aluminium, Silber und/oder Gold, metallbeschichtete Partikel, wie z.B. silberbeschichtetes Kupfer, silberbeschichtetes Nickel und/oder silberbeschichtete Glasmikrospären, anorganische Stoffe, wie z.B. Barium-Titantrioxid, Strontium-Titantrioxid, Siliziumdioxid, Aluminiumoxid, Zinkoxid, Titandioxid, Manganoxid, Kupferoxid, Antimonoxid, geschmolzenes Silizium, amorphes geschmolzenes Silizium, Ionenaustauscherharze, Lithium enthaltende Metalloxide, hohle Glasmikrospäre, Silizium-Solgel, Titan-Solgel und/oder gemischtes Titan, auf Kohlenstoff basierende Stoffe, wie z.B. Kohlenstoff, aktivierte Holzkohle und/oder Diamantpuder, Elastomere, wie z.B. Polybutadien und/oder Polysiloxan, Halbmetalle und/oder keramisches Material verwendet werden.

Der Gehalt an Absorptionsmittel(n) kann 10 bis 70 Gew. %, bezogen auf Gesamtgewicht der Mischung aus Polymer, Kanalbildner und Absorptionsstoffen, betragen.

Der Gehalt darf nicht zu hoch sein, da sonst die Deckschicht spröde wird. Ist der Gehalt zu gering, ist der Feuchtigkeitsschutz nicht ausreichend.

Die in das Polymer eingearbeiteten Absorptionsmittel sind gleichmäßig in dem Polymer bzw. der Folie verteilt. Die in das Polymer eingebrachten Kanalbildner bilden Kanäle, die die ganze Folie bzw. das aufgebrachte Muster von außen nach innen durchziehen. Sauerstoff, Feuchtigkeit oder andere unerwünschte Stoffe können somit von der äußeren Umgebung in den Folien- bzw. Musterinnenraum migrieren und mit den Absorptionsmittel im Inneren der Folie bzw. Muster reagieren. Die Absorptionsrate
ist um ein Vielfaches höher, da eine größere Anzahl an Absorptionsmittelteilchen mit den unerwünschten Stoffen reagieren kann als bei Abwesenheit der Kanalbildner. Je nach Wahl der Kanalbildner können feinere und damit eine größere Anzahl an Kanälen mit vermehrten Verzweigungen oder größere, weniger verzweigte Kanäle in kleinerer Anzahl im Polymer entstehen. Je feiner die Kanäle , desto größer ist die Absorptionsrate für unerwünschte Stoffe.

Falls erwünscht können noch pharmazeutisch unbedenkliche Farbstoffe in die Mischung aus Polymer, Kanalbildner und Absorptionsmittel gegeben werden.

Wenn die Deckschicht oder abziehbare Schutzschicht aus einer Folie mit integrierten Kanalbildnern und Absorptionsmitteln besteht, beträgt die Dicke der Schicht 5-100 µm, insbesondere 15-40 µm, bevorzugt 15 µm.

Für den Fall, daß die Deckschicht oder abziehbare Schutzschicht aus einer herkömmlichen Folie besteht, die entweder vollflächig oder unter Ausbildung eines Musters mit einer Mischung aus einem Polymer, Kanalbildnern und Absorptionsmitteln beschichtet ist, kann die Dicke beliebig eingestellt werden. Der einzige zu beachtende Punkt stellt die Flexibilität dar. Sie muß eine zufriedenstellende Nutzung des transdermalen therapeutischen Systems gewährleisten.

Die herkömmlich für eine wirkstoffundurchlässige Deckschicht (4) verwendete Folie kann erfindungsgemäß auf der Oberund/oder Unterseite mit der Mischung (5) beschichtet werden.

Die herkömmlich für die abziehbare Schutzschicht verwendete Folie kann erfindungsgemäß auf der nach außen gerichteten Seite mit der Mischung beschichtet werden.

Unter einem transdermalen therapeutischen System wird ein Pflaster verstanden. Bei diesem Pflaster kann es sich um ein Matrix- oder Membransystem handeln, welches eine wirkstoffundurchlässige Deckschicht (4) und eine abziehbare Schutzschicht (1) aufweist. Es können entweder beide Schichten mit den Kanalbildnern und Absorptionsstoffen versehen werden oder nur eine der Schichten.

Ist ein eigener Polymerträger vorgesehen, so können für die abziehbare Schutzschicht als herkömmliche Folien Polyester, Polyethylen, Polypropylen, Polysiloxan, Polyacrylat, Ethylenvinylacetat, Polyurethan, Polyisobuten oder Papier, meistens mit Silikon- und/oder Polyethylen beschichtet, oder ein Verbund aus diesen in Betracht kommen.

Ist ein eigener Polymerträger vorgesehen, so können die in herkömmlichen transdermalen therapeutischen Systemen verwendeten Deckschichten als Folien aus Acetal, Acrylat, Acrylonitril-Butadien-Styrol, Acrylonitril (Methyl Methacrylat) Copolymer, Acrylonitril Copolymer, Ethylen Ethyl Acrylat, Ethylen Methyl Acrylat, Ethylen Vinyl Acetat, Ethylen Vinyl Acetat Copolymer, Ethylen Vinylalkohol Polymer, Ionomere, Nylon (Polyamid), Nylon (Polyamid) Copolymer, Polybutylen, Polycarbonat, Polyester, Polyethylenterephthalat, thermoplastisches Polyester Copolymer, Polyethylen Copolymer (high density), Polyethylen (high-molecular-weight, high-density), Polyethylen (intermediate-molecular-weight, high-density), Polyethylen(linear low density), Polyethylen (low density), Polyethylen (medium density), Polyethylenoxid, Polyimid, Polypropylen, Polypropylen (coated), Polypropylen (oriented), Polystyrol, Polyurethan, Polyvinylacetat, Polyvinylchlorid, Polyvinylidenchlorid und/oder Styrol-Acrylonitril eingesetzt werden, die bei Bedarf metallisiert oder pigmentiert sein können.

Gemäß einer Ausführungsform besteht das Matrixpflaster aus der wirkstoffundurchlässigen Deckschicht (4), aus einer oder mehreren den Wirkstoff und/oder Permeationsförderer enthaltenden, selbstklebenden Matrixschicht(en) (2) oder einer oder mehreren Matrixschicht(en) (13), die mit einem Haftkleber (10) beschichtet sind und einer abziehbaren Schutzschicht (1).

Für die Matrix werden die medizinisch üblichen Matrixbildner wie Polyacrylat, Silikon, Polyisobutylen, Kautschuk, kautschukähnliche synthetische Homo-, Co- oder Blockpolymere, Butylkautschuk, Styrol/ Isopren-Copolymerisat, Polyurethane, Copolymere des Ethylens, Polysiloxane, Styrol/ Butadien- Copolymerisat oder ein Gemisch aus diesen, wie sie im Stand der Technik vorgesehen werden, verwendet.

Als Kleber kann Polydimethylsiloxan, Polyacrylate, Polyisobutylen, Polyacrylat mit C₄- C₁₀ Alkylalkoholestern, Polyurethan, Polyvinylether, amminrestistentes Silikon in Ethylacetat oder n-Heptan, Polyisobutylen/ Mineralöl oder ein Gemisch aus diesen verwendet werden.

Eine weitere erfindungsgemäße Ausführungsform stellt ein Membransystem dar. Dieses besteht aus der wirkstoffundurchlässigen Deckschicht (4), einem wirkstoffhaltigen Reservoir oder einer Reservoirschicht (12), einer semipermeablen Membran (11), einer fakultativen Haftklebeschicht (10) und einer abziehbaren Schutzschicht (1) .

Das Reservoir enthält den oder die Wirkstoff(e) und/oder Permeationsförderer, Stabilisatoren, Emulgatoren, Verdickungsmittel und/oder übliche Membransystem- bzw. Reservoirpflaster- Hilfsmittel. Das Reservoir bzw. die Reservoirschicht liegt zwischen der Deckschicht und der Membran. Als Gelbildner können bei Bedarf Methylcellulose, Hydroxypropylcellulose, Hydroxyethylcellulose, Carboxyvinylpolymer, Natrium- Plyoxilat, Carboxymethylcellulose oder ein Gemisch aus diesen verwendet werden.

Die Membran, die üblicherweise aus inerten Polymeren, insbesondere auf Basis von Polypropylen, Polyvinylacetat, Polyamid, Ethylen- Vinylacetat- Copolymeren und/oder Silikon, besteht, kann je nach Porengröße eine die Wirkstoffreisetzung kontrollierende Wirkung haben.

Für die Haftklebeschicht kann man ein druckempfindliches Klebemittel beispielsweise auf Polyurethanbasis, Polyisobutylenbasis, Polyvinyletherbasis, Siliconbasis, Polyacrylatbasis oder ein Gemisch aus diesen wählen.

Bei dem Klebemittel auf Silkonbasis kann es sich um Silikonkleber handeln, welche auf zwei Hauptbestandteilen basieren: Ein Polymer oder Klebstoff, insbesondere Polysiloxan, und ein die Klebrigkeit erhöhendes Harz. Der Polysiloxankleber ist gewöhnlich mit einem Vernetzer für den Kleber, typischerweise mit einem hochmolekularen Polydiorganosiloxan, und mit dem Harz zubereitet, um über ein angemessenes organisches Lösungsmittel eine dreidimensionale Silikatstruktur zu ergeben. Die Zumischung des Harzes zu Polymer ist der wichtigste Faktor, um die physikalischen Eigenschaften der polysiloxanen Kleber zu ändern; vgl. beispielsweise Sobieski, et al., "Silicone Pressure Sensitive Adhesives", Handbook of Pressure Sensitive Adhesive Technology, 2^{nd} ed., pp. 508-517 (D. Satas, ed.), Van Nostrand Reinhold, New sive Technology, 2^{nd} ed., pp. 508-517 (D. Satas, ed.), Van Nostrand Reinhold, New York (1989).

Ein weiteres Beispiel für ein druckempfindliches Klebemittel auf Silikonbasis ist trimethyliertes Siliciumdioxid, das mit Polydimethylsiloxan mit endständigen Trimethylsiloxy-Gruppen behandelt worden ist.

Bei den Klebemitteln auf Polyacrylatbasis kann es sich um ein beliebiges Homopolymer, Copolymer oder Terpolymer, bestehend aus verschiedenen Acrylsäurederivaten handeln.

So können die Polyacrylate Polymere eines oder mehrerer Monomere von Acrylsäuren und anderen copolymerisierbaren Monomeren sein. Außerdem können die Acrylatpolymere Copolymere von Alkylacrylaten und/oder -methacrylaten und/oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat- oder Alkylmethacrylat-Monomers, 0 bis 20 % eines funktionellen Monomers, copolymerisierbar mit Acrylat, und 0 bis 50 % eines anderen Monomeren.

Im folgenden sind verschiedene Acrylatmonomere, wie z.B. Acrylsäure, Methacrylsäure, Butylacrylat, Butylmethacrylat, Hexylacrylat, Hexylmethacrylat, Isooctylacrylat, Isooctylmethacrylat, Glycidylmethacrylat, 2-Hydroxyethylacrylat, Methylacrylat, Methylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat, aufgeführt, die alleine oder in Mischung polymerisiert werden können.

Zusätzlich können funktionelle Monomere, die mit den oben genannten Acrylaten copolymerisierbar sind, wie beispielsweise Acrylsäure, Methacrylsäure, Maleinsäure, Maleinanhydrid, Hydroxyethylacrylat, Hydroxypropylacrylat, Acrylamid, Dimethylacrylamid, Acrylnitril, Dimethylaminoethylacrylat, Dimethylaminoethylmethacrylat, tert.-Butylaminoethylacrylat, ter.-Butylaminoethylmethacrylat, Methoxyethylacrylat, Vinylacetat und Methoxythylmethacrylat, zur Copolymerisierung eingesetzt werden.

Weiter Einzelheiten und Beispiele für druckempfindliche Acrylate, welche für die Erfindung geeignet sind, sind in Satas Handbook of Pressure Sensitive Adhesive Technology "Acrylic Adhesives", 2^{nd} ed., pp. 396-456 (D. Satas, ed.), Van Nostrand Reinhold, New York (1989) beschrieben.

Der in dem transdermalen therapeutischen System enthaltene Wirkstoff kann z.B. ein Vertreter aus der Wirkstoffgruppe der Corticoide, Androgene, Östrogene, Gestagene, Protonenpumpenhemmer, 5-HT₁-Antagonisten, Sympatholytika/Sympathomimetika, Anticholinergika, Tranquillantien/Anxiolytika, Entwöhnungsmittel, Analgetika, Calcium-Antagonisten, Antiemetika, Vasodilatoren, Opiat-Antagonisten, Gerinnungshemmer, Antiparkinsonmittel, Antidementiva/Cholinesterasehemmer, ACE-Hemmer, Antihistaminika, Ulkustherapeutika/H₂-Rezeptorblocker, Angiotensin-II-Antagonisten, Neuroleptika, Antidepressiva, Lokälanästhetika und/oder Lipidsenker sein.

Das transdermale therapeutische System kann einen oder mehrere Vertreter aus der Gruppe der Corticoide z. B. Beclomethason, Budesonidpropionat, Flunisolidacetat, Triamcinolon, Fluticason, Betamethason-17-valerat, Glycinsäure, Fluocortolon, Beclomethasondipropionat, Budesonidbase, Dexamethason, Hydrocortison, Flunisolid, Prednison, Triamcinolonacetonid, Methylprednisolon, Betamethason, Deflazacort, Cortison, Cortisonacetat, Prednyliden, Cloprednol, Fluocortolon-21-hexanoat, Prednicarbat und/oder deren Derivate und/oder deren pharmazeutisch unbedenklichen Salze als Wirkstoffkomponente enthalten.

Das transdermale therapeutische System kann einen oder mehrere Vertreter aus der Gruppe der Androgene z.B. Testosteron, Testosteronundecanoat, Androsteron und/oder deren Derivate und/oder deren pharmazeutisch unbedenklichen Salze als Wirkstoffkomponente enthalten.

Das transdermale therapeutische System kann einen oder mehrere Vertreter aus der Gruppe der Östrogene z.B. Estradiol, Estradiolbenzoat, Estradiolvalerat, Estradioldipropionat, Estron, Estriol, Ethinylestradiol, Diethylstilbestrol, Diethylstilbestroldimethylether, Diethylstilbestroldiphosphat, Diethylstilbestroldipropionat und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Gestagene z.B. Progesteron, Cyproteronacetat, Cyproteron, Chlormadinon, Chlormadinonacetat, Medroxyprogesteronacetat, Levonorgestrel, Norgestrel, Norgestimat, Norethiestronacetat und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Protonenpumpenhemmer z.B. Omeprazol, Esomeprazol, Lansoprazol, Leminoprazol, Pantoprazol, Rabeprazol, Polaprezinc und/oder deren Derivate und/oder deren pharmazeutisch unbedenklichen Salze als Wirkstoffkomponente enthalten.

Das transdermale therapeutische System kann einen oder mehrere Vertreter aus der Gruppe der Migränemittel bzw. 5-HT₁-Antagonisten z.B. Lisurid, Sumatriptan, Sumatriptanhydrogensuccinat, Rizatriptan, Rizatriptanbenzoat, Almotriptan, Avitriptan, Eletriptan, Frovatriptan, Naratriptan, Zolmitriptan und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Sympatholytika/Sympathomimetika z.B. Adimolol, Adrenalin, Albuterol, Alpenolol, Amosulalol, Arotinolol, Atenolol, Bambuterol, Betaxolol, Bevantolol, Bisoprolol, Bitolterol, Bopindolol, Broxaterol, Bucindolol, Bucumolol, Bufuralol, Bunitrolol, Bupranolol, Butofilolol, Carazolol, Carbuterol, Carteolol, Carvedilol, Cetamolol, Cicloprolol, Clenbuterol, Cloranolol, Crateolol, Dihydroergotamin, Dihydroergotamintartrat, Dihydroergotaminmesylat, Dilevalol, Doxazosin, Etilefrin, Epanolol, Esatenolol, Esmolol, Fenetyllin, Fenoterol, Formoterol, Ibuterol, Isoprenalin, Labetalol, Landiolol, Levobetaxolol, Levobunolol, Levosalbutamol, Mabuterol, Mepindolol, Metipranolol, Metoprolol, Morazon, Nebivolol, Nipradilol, Norfenefrin, Noradrenalin, Oxprenolol, Penbutolol, Picumeterol, Pimolol, Pindolol, Pirbuterol, Phenmetrazin Phenylephedrin, Phentolamin, Phenoxybenzamin, Prazosin, Procaterol, Propanolol, Rimiterol, Reproterol, Salbutamol, Salmeterol, Sotalol, Sulfonterol, Terazosin, Terbutalin, Tertatolol, Tienoxolol, Tilisolol, Timolol, Tolazolin, Toliprolol, Tolubuterol, Tamsulosin, Clonidin, Moxonidin und/oder deren Derivate und/oder deren pharmazeutisch unbedenklichen Salze als Wirkstoffkomponente enthalten.

Das transdermale therapeutische System kann einen oder mehrere Vertreter aus der Gruppe der Anticholinergika z.B. Ipratropium, Oxitropium, Atropin, Scopolaminbase, Ipratropiumbromid, Oxitropiumbromid, Atropinmethylbromid, Atropinmethylnitrat, Atropinsulfat, Atropinvalerianat, Scopolaminhydrobromid, Scopolaminhydrochlorid, Scopolaminhydroiodid, Tropicamid, Oxybutinin und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Tranquillantien/Anxiolytika z.B. Alprazolam, Bentazepam, Bromazepam, Camazepam, Clorazepat, Clonazepam, Clotiazepam, Diazepam, Etiracetam, Etiolam, Fludiazepam, Flunitrazepam, Flurazepam, Flutazolam, Flutoprazepam, Halazepam, Ketazolam, Loprazolam, Lorazepam, Lormetazepam, Medazepam, Metaclazapam, Mexazolam, Midazolam, Nitrazepam, Norazepam, Oxazepam, Oxazolam, Prazepam, Temazepam, Triazolam und/oder deren Derivate und/oder deren pharmazeutisch unbedenklichen Salze als Wirkstoffkomponente enthalten.

Das transdermale therapeutische System kann einen oder mehrere Vertreter aus der Gruppe der Entwöhnungsmittel z.B. Nicotin, Methadon, Disulfiram, Lobelin und/oder deren Derivate und/oder deren pharmazeutisch unbedenklichen Salze als Wirkstoffkomponente enthalten.

Das transdermale therapeutische System kann einen oder mehrere Vertreter aus der Gruppe der Analgetika z.B. Ibuprofen, Ketoprofen, Alminoprofen, Bermoprofen, Carprofen, Dexibuprofen, Dexketoprofen, Fenoprofen, Flobufen, Flunoxaprofen, Flurbiprofen, Loxoprofen, Pelobiprofen, Pranoprofen, Pentazocin, Tilnoprofen, Ximoprofen, Zaltroprofen, Diclofenac, Amfenac, Bromfenac, Clidanac, Etodolac, Felbinac, Fentiazac, Mofezolac, Oxindanac, Tifurac, Indomethacin, Acemetacin, Piroxicam, Ampiroxicam, Meloxicam, Isoxicam, Lornoxicam, Tenoxicam, Butorphanol Buprenorphin, Morphin, Hydromorphon, Dihydrocodein, Oxycodon, Piritramid, Pentazocin, Levomethadon, Tramadol, Fentanyl, Codein, Codeinhydrochlorid, Codeinphosphat, Tilidin, Tilidinmesylat, Tilidinhydrochlorid, Diclofenac-Natrium, Amfenac-Natrium, Bromfenac-Natrium, Clidanac-Natrium, Etodolac-Natrium, Felbinac-Natrium, Fentiazac-Natrium, Mofezolac-Natrium, Oxindanac-Natrium, Tifurac-Natrium, Indomethacin-Natrium, Acemetacin-Natrium, Meloxicam-Cyclodextrin, Buprenorphinhydrochlorid, Morphinacetat, Hydromorphonhydrochlorid, Oxycodonhydrochlorid, Piritramidhydrogentartrat, Levomethadonhydrochlorid, Fentanyldihydrogencitrat und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Calcium-Antagonisten z.B. Amlodipin, Arandipin, Azelmidipin, Barnidipin, Benidipin, Cilnidipin, Efonidipin, Felodipin, Flordipin, Iganidipin, Isradipin, Lacidipin, Lercanidipin, Manidipin, Nicardipin, Nifedipin, Nilvadipin, Nisoldipin, Nitrendipin, Palonidipin, Pranidipin, Ticlopidin, Vatanidipin, Clentiazem und/oder deren Derivate und/oder deren pharmazeutisch unbedenklichen Salze als Wirkstoffkomponente enthalten.

Das transdermale therapeutische System kann einen oder mehrere Vertreter aus der Gruppe der Antiemetika z.B. Alizaprid, Azasetron, Batanoprid, Cleboprid, Dazoprid, Dolasetron, Domperidon, Granisetron, Itasetron, Levosulpirid, Metoclopramid, Nabilon, Ondansetron, Pancoprid, Ramosetron, Tropisetron, Zatosetron und/oder deren Derivate und/oder deren pharmazeutisch unbedenklichen Salze als Wirkstoffkomponente enthalten.

Das transdermale therapeutische System kann einen oder mehrere Vertreter aus der Gruppe der Vasodilatoren z.B. Glyceroltrinitrat (Nitroglycerin), Isosorbiddinitrat, Isosorbid-5-Mononitrat, Pentaerythrityltetranitrat, Molsidomin und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Opiat-Antagonisten z. B. Naloxon, Naltrexon und/oder deren Derivate und/oder deren pharmazeutisch unbedenklichen Salze als Wirkstoffkomponente enthalten.

Das transdermale therapeutische System kann einen oder mehrere Vertreter aus der Gruppe der Gerinnungshemmer z.B. Heparin-Natrium, Certoparin, Dalteparin, Danaparoid, Enoxaparin, Nadroparin, Reviparin, Tinzaparin, Heparinoide, Warfarin, Phenprocoumon, Acenocoumarol und/oder deren Derivate und/oder deren pharmazeutisch unbedenklichen Salze als Wirkstoffkomponente enthalten.

Das transdermale therapeutische System kann einen oder mehrere Vertreter aus der Gruppe der Antiparkinsonmittel z.B.. Aptiganel, Biperiden, Budipin, Cabergolin, Droxidopa, Etacapon, Idazoxan, Lazabemid, Milacemid, Mofegilin, Pergolid (Pergolidmesylat, Pergolidhydrochlorid), Pramipexol, Quineloran, Rasagelin, Remacemid, Ropinorol, Selegilin, Talipexol, Tolcapon und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Antidementiva/Cholinesterasehemmer z.B. Rivastigmin, Neostigmin, Physostigmin, Pyridostigmin, Donepezil, Tacrin und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der ACE-Hemmer z.B. Alacepril, Benazepril, Captopril, Ceronapril, Cilazapril, Denapril, Enalapril, Enalaprilmaleat, Fosinopril, Imidapril, Lisinopril, Moexipril, Moveltipril, Perindopril, Quinapril, Ramipril, Ramiprilat, Ramiprilmesylat, Rentiapril, Spirapril, Temocapril, Trandolapril, Trandolaprilmesylat, Utibapril, Zofenopril und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Antihistaminika z.B. Acrivastin, Astemizol, Carebastin, Cetirizin, Descarbethoxyloratadin, Dimetinden, Ebastin, Emedastin, Epinastin, Fexofenadin, Ketotifen, Levocabastin, Loratadin, Mequitazin, Mizolastin, Nafamostat, Norastemizol, Olopatidin, Oxatomid, Rupatadin, Tazifyllin, Temelastin, Traxanox und/oder deren Derivate und/oder deren weitere pharmazeutisch unbedenklichen Salze als Wirkstoffkomponente enthalten.

Das transdermale therapeutische System kann einen oder mehrere Vertreter aus der Gruppe der Ulkustherapeutika/ H₂-Rezeptorblocker z.B. Dalcotidin, Famotidin, Lafutidin, Niperdidin, Nizatridin, Osutidin, Pibutidin, Pirenzepin, Ramixotidin, Ranitidin, Proglumid, Misoprostol und/oder deren Derivate und/oder deren pharmazeutisch unbedenklichen Salze als Wirkstoffkomponente enthalten.

Das transdermale therapeutische System kann einen oder mehrere Vertreter aus der Gruppe der Angiotensin-II-Antagonisten z.B. Candesartan, Candesartan-Cilexetil, Losartan, Tasosartan, Telmisartan, und/oder deren Derivate und/oder deren pharmazeutisch unbedenklichen Salze als Wirkstoffkomponente enthalten.

Das transdermale therapeutische System kann einen oder mehrere Vertreter aus der Gruppe der Neuroleptika z.B. Sulpirid, Promethazin, Benperidol, Haloperidol, Chlorprothixen, Clozapin, Fluphenazin, Perphenazin, Droperidol, Pipamperon, Prothipendyl, Melperon, Flupentixoldecanoat, Fluspirilen, Bromperidol, Levomepromazinhydrogenmaleat, Zotepin, Pimozid, Perazin, Chlorprometazin, Trifluprometazin, Risperidon, Sertindol, Amisulprid, Olanzapin, Zuclopenthixol, Thioridazin und/oder deren Derivate und/oder deren pharmazeutisch unbedenklichen Salze als Wirkstoffkomponente enthalten.

Das transdermale therapeutische System kann einen oder mehrere Vertreter aus der Gruppe der Antidepressiva z.B. Amitriptylin, Clomopramin, Maprotilin, Doxepin, Citalopram, Fluvoxamin, Reboxetin, Alprazolam, Fluoxetin, Lofepramin, Mianserin und/oder deren Derivate und/oder deren pharmazeutisch unbedenklichen Salze als Wirkstoffkomponente enthalten.

Das transdermale therapeutische System kann einen oder mehrere Vertreter aus der Gruppe der Lokalanästhetika z.B. Lidocain, Prilocain, Benzocain, Cocain, Procain, Tetracain, Bupivacain, Cinchocain, Etidocain, Mepivacain, Butanilicain, Levobupivacain, Ropivacain und/oder deren Derivate und/oder deren pharmazeutisch unbedenklichen Salze als Wirkstoffkomponente enthalten.

Das transdermale therapeutische System kann einen oder mehrere Vertreter aus der Gruppe der Lipidsenker z.B. Colestyramin, Xantinolnicotinat, Fluvastatin, Simvastatin, Atorvastatin, Pravastatin, Cerivastatin, Dalvastatin, Itavastatin, Lovastatin, Dextrothyroxim-Natrium und/oder deren Derivate und/oder deren pharmazeutisch unbedenklichen Salze als Wirkstoffkomponente enthalten.

Der in dem transdermalen therapeutischen System beinhaltete Wirkstoff kann aber auch z.B. Leflunomid, Indapamid, Hydroxytamoxifen, Fusidinsäure, Finasterid, Tirofiban, Rosiglitazon, Pioglitazon, Montelukast und/oder deren Derivate und/oder deren pharmazeutisch unbedenklichen Salze sein.

Unter pharmazeutisch unbedenklichen Salzen der genannten Wirkstoffe werden Säureadditionssalze verstanden. Diese erhält man durch die Reaktion des in der freien Form vorliegenden Wirkstoffes mit pharmazeutisch unbedenklichen Säuren. Pharmazeutisch unbedenkliche Säuren sind anorganische Säuren (z.B. Salzsäure, Bromwasserstoffsäure, Schwefelsäure, Salpetersäure, Phosphorsäure) oder organische Säuren (z.B. Essig-, Propion-, Hydroxyessig-, Milch-, Brenztrauben-, Oxal-, Malein-, Malon-, Bernstein-, Fumar-, Äpfel-, Wein-, Citronen-, Methansulfon-, Ethansulfon-, Benzolsulfon-, p-Toluolsulfon-, Cyclohexansulfamin-, Salicyl-, p-Aminosalicyl- und Pamoasäure). Ebenso als Säureadditionssalze werden Solvate mit dem Wirkstoff bezeichnet. Derartige Solvate sind z.B. Hydrate, Alkoholate und dergleichen.
Als weitere mögliche pharmazeutisch unbedenkliche Salze der genannten Wirkstoffe kommen vor allem Alkalimetall- und/oder Erdalkalimetallsalze sowie das Ammoniumsalz in Frage wie z.B. das Kalium-, Natrium-, Lithium-, Calcium-, Magnesiumund Ammoniumsalz.

Als Permeationsförderer lassen sich gegebenenfalls einund/oder mehrwertige aliphatische, cycloaliphatische und/oder aromatisch- aliphatische Alkohole mit jeweils bis zu acht C- Atomen, z.B. Ethanol, 1,2-Propandiol, Dexpanthenol und/oder Polyethylenglykol; Alkohol/ WasserGemische; gesättigte und/oder ungesättigte Fettalkohole mit jeweils 8- 18 C- Atomen; Terpene; z.B. Cineol, Carveol, Menthon, Terpineol, Verbenon, Menthol, Limonen, Thymol, Cymen, Terpinen-4-ol, Neomenthol, Geraniol, Fenchon; Gemische aus Terpenen und Etanol und/oder Propylenglykol; Teebaumöl; gesättigte und/oder ungesättigte cyclische Ketone; Alkyl- Methylsulfoxide; gesättigte und/oder ungesättigte Fettsäuren mit jeweils 8- 18 C Atomen; deren Ester und Salze; natürliches Vitamin E; synthetisches Vitamin E und/oder Vitamin E- Derivate; Sorbitanfettsäureester und ethoxylierte Sorbitanfettsäureester; Azone (Laurocapram); Azone gemischt mit Alkoholen; Harnstoff; 1-Alkylpyrrolidon; Blockcopolymere von Polyethylenglykol und Dimethylsiloxan mit kationischer Gruppe an einem Ende; Isopropylmyristat, Isopropylpalmitat, Folat-Polyethylenglykol-Liposom, Proliposom; Polyoxyethylen-10-stearylether; Gemisch aus Polyoxyethylen-10-stearylether und Glyceryldilaurat; Dodecyl-2-(N,N-dimethylamino)-propanoltetradecanoat und/oder Dodecyl-2-(N,N-dimethylamino)-propianat; N-Acetylprolinatester mit > 8 C-Atomen; nichtionische Tenside, z.B. Laurylether, Ester von Polyoxyethylen; Ethosom (Phospholipidvesikel); Dimethyl(arylimino)sulfuran; Gemisch aus Ölsäureanaloga und Propylenglykol; Gemisch aus Padimat O, Oktylsalicylat, Oktylmethoxycinnimat, Laurocapram; hochdisperses Siliziumdioxid (Aerosil®); Polyoxyethylen-7-glycerolmonococoat (Cetiol® HE); 2-Octyldodecanol (Eutanol® G) oder ein Gemisch aus Einzelkomponenten verwenden.
**Figur 1** zeigt die Kaschierung der Deckfolie (3) bestehend aus Polymer, Kanalbildner und Absorptionsmittel auf das Laminat, das die selbstklebende Klebstoffmatrix (2) und die abziehbare Schutzschicht (1) enthält. Dabei wird das Laminat abgerollt (6) und mit Hilfe der Kaschierwalze (7) auf das Laminat aufgetragen.
**Figur 2** zeigt die Auftragung der Mischung (5), bestehend aus Polymer, Kanalbildner und Absorptionsmittel, mit einem Walzenauftragsystem (8) auf die Oberseite der Deckfolie (4) sowie die anschließende Kaschierung mit der Kaschierwalze (7) der nun partiell mit der Mischung (5) beschichteten Deckfolie (4) auf das Laminat, das die selbstklebende Klebstoffmatrix (2) und die abziehbare Schutzschicht (1) enthält.
**Figur 3** zeigt die Auftragung der Mischung (5), bestehend aus Polymer, Kanalbildner und Absorptionsmittel, mit einem Walzenauftragsystem (8) auf die Oberseite der Deckfolie (4), die ein Bestandteil des Laminates ist, das zudem die selbstklebende Klebstoffmatrix (2) und die abziehbare Schutzschicht (1) enthält. Im Anschluß an den Auftrag der Mischung erfolgt die Stanzung der Pflaster (9) und das Abgittern des Überstandes.
**Figur 4** zeigt die Auftragung der Mischung (5), bestehend aus Polymer, Kanalbildner und Absorptionsmittel, mit einem Walzenauftragsystem (8) auf die Unterseite der Deckfolie (4) sowie die anschließende Kaschierung mit der Kaschierwalze (7) der nun partiell mit der Mischung (5) beschichteten Deckfolie (4) auf das Laminat, das die selbstklebende Klebstoffmatrix (2) und die abziehbare Schutzschicht (1) enthält.
**Figur 5** zeigt die Auftragung der Mischung (5), bestehend aus Polymer, Kanalbildner und Absorptionsmittel, mit einem Walzenauftragsystem (8) auf die nach außen gerichtete Seite der abziehbaren Schutzschicht (1), die ein Bestandteil des Laminates ist, das zudem die selbstklebende Klebstoffmatrix (2) und die Deckschicht (4) enthält. Im Anschluß an den Auftrag der Mischung erfolgt die Stanzung der Pflaster (9) und das Abgittern des Überstandes.
**Figur 6** zeigt eine Draufsicht auf das Laminat, das aus der Deckschicht (3), die mindestens ein Polymer, Kanalbildner und Absorptionsmittel enthält, der selbstklebenden Klebstoffmatrix (2) und der abziehbaren Schutzschicht (1) besteht und die verschiedenen Herstellungsschritte bis zum verpackten transdermalem therapeutischen System. Im Schritt A wird die Kontur des transdermalen therapeutischen Systems aus dem Laminat gestanzt. Schritt B zeigt die Entfernung des Überstandes. In Schritt C wird die Außenkontur des transdermalen therapeutischen Systems gestanzt. Schritt D zeigt das transdermale therapeutische System nach dem Abgittervorgang. E zeigt das transdermale therapeutische System mit aufgestanzter Abziehhilfe in dem Verpackungssachet (Durchsicht).
**Figur 7** zeigt einen Querschnitt der erfindungsgemäßen Deckschicht (3) bzw. abziehbaren Schutzschicht (1) eines transdermalen therapeutischen Systems. Diese Deckschicht (3) bzw. abziehbare Schutzschicht (1) wird durch ein Polymer (16) gebildet, in dem die eingearbeiteten Kanalbildner eine Vielzahl von verzweigten Kanälen (14) bilden. Außerdem sind in dem Polymer Absorptionsmittelteilchen (15) gleichmäßig verteilt.
**Figur 8** zeigt den Querschnitt durch ein Membransystem, welches aus der wirkstoffundurchlässigen Deckschicht (4), die mit der Mischung aus Polymer, Kanalbildner und Absorptionsmittel (5) partiell beschichtet ist, einem die Wirkstoffe enthaltenden Reservoir oder einer Reservoirschicht (12), einer für die Wirkstoffe durchlässigen Membran (11), einer Haftklebeschicht (10) und einer dem Stand der Technik entsprechenden abziehbaren Schutzschicht (1) besteht.
**Figur 9** zeigt den Querschnitt durch ein Membransystem, welches aus der die Kanalbildner und Absorptionsmittel enthaltenden wirkstoffundurchlässigen Deckschicht (3), der herkömmlichen Deckschicht (4), einem die Wirkstoffe enthaltenden Reservoir oder einer Reservoirschicht (12), einer für die Wirkstoffe durchlässigen Membran (11), einer Haftklebeschicht (10) und einer dem Stand der Technik entsprechenden abziehbaren Schutzschicht (1) besteht.
**Figur 10** zeigt den Querschnitt durch ein Matrixsystem, welches aus der die Kanalbildner und Absorptionsmittel enthaltenden wirkstoffundurchlässigen Deckschicht (3), einer die Wirkstoffe und gegebenenfalls Permeationsförderer enthaltenden Matrixschicht (13), einer Haftklebeschicht (10) und einer dem Stand der Technik entsprechenden abziehbaren Schutzschicht (1) besteht.
**Figur 11** zeigt den Querschnitt durch ein Matrixsystem, welches aus der erfindungsgemäßen wirkstoffundurchlässigen Deckschicht (4), die mit der Mischung aus Polymer, Kanalbildner und Absorptionsmittel (5) partiell beschichtet ist, einer die Wirkstoffe und gegebenenfalls Permeationsförderer enthaltenden Matrixschicht (13), einer Haftklebeschicht (10) und einer dem Stand der Technik entsprechenden abziehbaren Schutzschicht (1) besteht.

Die Erfindung wird zudem durch nachstehende Beispiele näher erläutert ohne aber den Erfindungsumfang damit einzuschränken.

### Beispiel 1:

Herstellung einer erfindungsgemäßen wirkstoffundurchlässigen Deckschicht oder abziehbaren Schutzschicht eines transdermalen therapeutischen Systems in Form einer Folie:

Zunächst werden die Komponenten Polymer, Kanalbildner und Absorptionsmittel bei erhöhter Temperatur gemischt, wobei bevorzugt eine Vormischung aus Polymer und Kanalbildner erfolgt.

Die Mischung wird bis zur Schmelze erhitzt und über gängige Verfahren (Stand der Technik) im Gieß- oder Blasextrusionsverfahren zu einer Folie verarbeitet. Während der Verarbeitung kann es erforderlich sein, den Herstellungsbereich bezüglich der umgebenden Atmosphäre partiell oder vollständig zu konditionieren (v.a. reduzierte r.F., reduzierter 02-Gehalt) beispielsweise durch Einsatz getrockneter Schutzgase. Im Anschluß an diesen Herstellvorgang können ggf. zusätzliche Reckungen der Folien zur Modifizierung der mechanischen Eigenschaften und zur Reduzierung der Dicke vorgenommen werden. Dieser Herstellvorgang kann dem Stand der Technik entnommen werden.

Die so erhaltene Folie kann in geeigneter Weise gelagert werden. Sie dient zum Abdeckung einer haftklebend ausgerüsteten wirkstoffhaltigen Schicht, die bereits einseitig mit einem trennbeschichteten bzw. mit einer Trennbeschichtung versehenen Trägermaterial abgedeckt ist. Ggf. kann dieses Material zunächst auch beidseitig mit trennbeschichteten Trägermaterialien versehen sein, wobei eine Trägerschicht unmittelbar vor dem Zusammenfügen mit der o.g. Folie entfernt werden muß (umkaschieren). Weiterhin kann die Zwischenschicht aus mehreren Lagen bestehen, wobei mindestens eine dieser Lagen Wirkstoffe enthält. So wird ein Laminat aus trennbeschichteter Folie, wirkstoffhaltiger Schicht oder Schichten und trockenmittelhaltiger Schicht erhalten. Aus diesem Laminat wird durch Stanzen, Schneiden oder andere geeignete Verfahren eine Einzeldosierung erzeugt, die unter geeigneten Bedingungen zwischengelagert oder direkt verpackt wird. Auch hierbei kann es während der Verarbeitung oder der Zwischenlagerung erforderlich sein, den Herstellungsbereich bezüglich der umgebenden Atmosphäre partiell oder vollständig zu konditionieren (v.a. reduzierte r.F., reduzierter 02-Gehalt), beispielsweise durch Einsatz getrockneter Schutzgase.

### Beispiel 2:

Herstellung einer mit einem Absorptionsmittel und Kanalbildner beinhaltendem Polymerträger beschichteten wirkstoffuridurchlässigen Deckschicht oder abziehbare Schutzschicht eines transdermalen therapeutischen Systems

Zunächst werden die Komponenten Polymer, Kanalbildner und Absorptionsmittel bei erhöhter Temperatur gemischt, wobei bevorzugt eine Vormischung aus Polymer und Kanalbildner erfolgt.

Die erhaltene Mischung wird portionsweise für eine spätere Weiterverarbeitung verpackt oder direkt weiterverarbeitet. Die Mischung wird dazu aufgeschmolzen z.B. in einem Extruder oder über andere geeignete Misch- und/oder Dosiergeräte, wie sie beispielsweise in den Grundvarianten im Heißschmelzkleberauftrag verbreitet sind. Im Anschluß wird die Mischung z.B. durch Düsen- oder Walzenauftrag auf eine Folie, die bereits Teil des wirkstoffhaltigen Verbundes ist oder wird, definiert in Menge und/oder Position aufgebracht. Bevorzugt wird eine Dosierung auf die wirkstoffundurchlässige Deckschicht eines wirkstoffhaltigen Verbundes unmittelbar vor der Herstellung und Verpackung der Einzeldosierung. Auch hierbei kann es während der Verarbeitung oder der Zwischenlagerung erforderlich sein, den Herstellungsbereich bezüglich der umgebenden Atmosphäre partiell oder vollständig zu konditionieren (v.a. reduzierte r.F., reduzierter 02-Gehalt), beispielsweise durch Einsatz getrockneter Schutzgase.

### Beispiel 3:

| Zusammensetzung der Mischung: | |
|---|---|
| Polypropylen | 70 Gew.% |
| Polyethylenglykol | 10 Gew.% |
| Molekularsieb 4Å | 20 Gew.% |

### Beispiel 4:

| Zusammensetzung der Mischung: | |
|---|---|
| Polypropylen | 35 Gew.% |
| Polyethylenglykol | 12 Gew.% |
| Molekularsieb 4Å (Baylith® T Pulver) | 53 Gew.% |

### Beispiel 5:

| Zusammensetzung der Mischung: | |
|---|---|
| Polyethylen | 55 Gew.% |
| Polyethylenglykol | 10 Gew.% |
| Molekularsieb 4Å | 35 Gew.% |

## Patentansprüche

1. Eine wirkstoffundurchlässige Deckschicht und/oder eine abziehbare Schutzschicht eines transdermalen therapeutischen Systems, wobei die Deckschicht und/oder Schutzschicht **gekennzeichnet ist durch** eine Mischung aus mindestens einem Polymer und mindestens einem eingebettetem Absorptionsmittel und mindestens einem eingebetteten Kanalbildner.

2. Eine wirkstoffundurchlässige Deckschicht in Form einer Folie und/oder eine abziehbare Schutzschicht in Form einer Folie eines transdermalen therapeutischen Systems, **dadurch gekennzeichnet, daß** die Folie mit einer Mischung aus mindestens einem Polymer, mindestens einem Kanalbildner und mindestens einem Absorptionsmittel vollflächig oder in Mustern beschichtet ist.

3. Eine wirkstoffundurchlässige Deckschicht und/oder eine abziehbare Schutzschicht nach Anspruch 1 oder 2, **gekennzeichnet durch** ein Thermoplast oder ein vernetzbares bzw. vernetztes Polymer als Polymer der Mischung aus Polymer, Absorptionsmittel und Kanalbildner.

4. Eine wirkstoffundurchlässige Deckschicht und/oder eine abziehbare Schutzschicht nach Anspruch 2 oder 3, **dadurch gekennzeichnet, daß** die Deckschicht und/oder die Schutzschicht mit einer Mischung (Polymerträger) aus mindestens einem Thermoplast, mindestens einem Kanalbildner und mindestens einem Absorptionsmittel beschichtet worden ist.

5. Eine wirkstoffundurchlässige Deckschicht und/oder eine abziehbare Schutzschicht nach Anspruch 2 oder 3, **dadurch gekennzeichnet, daß** die Deckschicht und/oder die Schutzschicht mit einer Mischung (Polymerträger)
(i) aus mindestens einem thermisch vernetzbaren Polymeren, mindestens einem Kanalbildner und mindestens einem Absorptionsmittel kalt oder in der Wärme beschichtet worden ist oder
(ii) aus mindestens einem durch Strahlung, insbesondere UV-Strahlung vernetzbaren Polymeren, mindestens einem Kanalbildner und mindestens einem Absorptionsmittel kalt oder in der Wärme beschichtet worden ist und
jeweils danach die Vernetzung durchgeführt worden ist.

6. Eine wirkstoffundurchlässige Deckschicht und/oder eine abziehbare Schutzschicht eines transdermalen therapeutischen Systems nach einem der vorhergehenden Ansprüche, **gekennzeichnet dadurch, daß** das Polymer ein Polyolefin, Polyisopren, Polybutadien, Polybuten, Polysiloxan, Polyamid, Ethylen-Vinylacetat-Copolymer, Ethylen-Methacrylat-Copolymer, Polystyrol, Polyester, Polyanhydrid, Polyacrylatnitril, Polyacrylnitril, Polysulfonat, Polyesteramid, Polyacrylatester, Propylen-Maleinsäureanhydrid, Polyethylen-Maleinsäureanhydrid, Polyethylen-Urethan, Polyethylen-Ethylvinylalkohol, Polyethylen-Nylon und/oder Polyurethan ist.

7. Eine wirkstoffundurchlässige Deckschicht und/oder eine abziehbare Schutzschicht nach Anspruch 6, **gekennzeichnet durch** Polyethylen und/oder Polypropylen als Polymer.

8. Eine wirkstoffundurchlässige Deckschicht und/oder eine abziehbare Schutzschicht eines transdermalen therapeutischen Systems nach einem der vorhergehenden Ansprüche, **gekennzeichnet durch** einen Polymergehalt von 10 bis 90 Gew. % bezogen auf das Gesamtgewicht der Mischung aus Polymer, Kanalbildner und Absorptionsmittel.

9. Eine wirkstoffundurchlässige Deckschicht und/oder eine abziehbare Schutzschicht eines transdermalen therapeutischen Systems nach einem der vorangegangenen Ansprüche, **gekennzeichnet durch** hydrophile Stoffe als Kanalbildner.

10. Eine wirkstoffundurchlässige Deckschicht und/oder eine abziehbare Schutzschicht eines transdermalen therapeutischen Systems nach Anspruch 9, **gekennzeichnet durch** Polyglykole, Ethylvinylalkohole, Glycerin, Pentaerithritol, Polyvinylalkohole, Polyvinylpyrrolidon, Vinylpyrrolidon, N-Methylpyrrolidon, Polysaccharide, Saccharide und/oder zuckeralkohole als Kanalbildner.

11. Eine wirkstoffundurchlässige Deckschicht und/oder eine abziehbare Schutzschicht nach Anspruch 10, **gekennzeichnet durch**
- Polyethylenglykol und/oder Polypropylenglykol als Polyglykol und/oder
- Glucose, Mannose, Galactose und/oder Fructose als Saccharid und/oder
- Mannitol, Sorbitol, Hexitol, Dulcitol, Xylitol, Ribitol und/oder Erythrol als Zuckeralkohol.

12. Eine wirkstoffundurchlässige Deckschicht und/oder eine abziehbare Schutzschicht eines transdermalen therapeutischen Systems nach Anspruch 9, 10 oder 11, **gekennzeichnet durch** einen Kanalbildnergehalt von 10 bis 40 Gew. % bezogen auf das Gesamtgewicht der Mischung aus Polymer, Kanalbildner und Absorptionsmittel.

13. Eine wirkstoffundurchlässige Deckschicht und/oder eine abziehbare Schutzschicht nach einem der vorhergehenden Ansprüche, **dadurch gekennzeichnet, daß** es sich bei dem Absorptionsmittel um ein derartiges Mittel zur Absorption von Feuchtigkeit, Sauerstoff oder von Stoffen handelt, die von einem transdermalen therapeutischen System bei Lagerung abgegeben werden, besonders von geruchsintensiven Stoffen, insbesondere von Aminen oder Polymermonomeren.

14. Eine wirkstoffundurchlässige Deckschicht und/oder eine abziehbare Schutzschicht eines transdermalen therapeutischen Systems nach einem der vorangegangenen Ansprüche, **gekennzeichnet durch** Trockenmittel, Metalle, Legierungen, metallbeschichtete Partikel, anorganische Stoffe, Ionenaustauscherharze, auf Kohlenstoff, insbesondere auf elementarem Kohlenstoff, basierende Stoffe, Elastomere, Halbmetalle und/oder keramisches Material als Absorptionsmittel.

15. Eine wirkstoffundurchlässige Deckschicht und/oder eine abziehbare Schutzschicht eines transdermalen therapeutischen Systems nach Anspruch 14, **gekennzeichnet dadurch, daß** die physikalischen Trockenmittel Molekularsiebe, Siliziumgele, Erden, synthetische Polymere und/oder Stärken, insbesondere Molekularsiebe, umfassen.

16. Eine wirkstoffundurchlässige Deckschicht und/oder eine abziehbare Schutzschicht nach Anspruch 15, **gekennzeichnet durch** Montmorrillimiterde oder Montmorrilloniterde oder Polymere, die in Babywindeln verwendet werden, oder Molekularsiebe mit einer Porengröße von 3 bis 15 Angström oder Siliziumdioxidgel einer Porengröße von etwa 24 Angström als Trockenmittel.

17. Eine wirkstoffundurchlässige Deckschicht und/oder eine abziehbare Schutzschicht nach Anspruch 14, **gekennzeichnet durch**
- Nickel, Kupfer, Aluminium, Silber und/oder Gold als Metall oder Legierung,
- silberbeschichtetes Kupfer, silberbeschichtetes Nickel und/oder silberbeschichtete Glasmikrosphären als metallbeschichtete Partikel,
- Barium-Titantrioxid, Strontium-Titantrioxid, Siliziumdioxid, Aluminiumoxid, Zinkoxid, Titandioxid, Manganoxid, Kupferoxid, Antimonoxid, geschmolzenes oder erschmolzenes Silizium, amorphes geschmolzenes oder amorphes erschmolzenes Silizium, Ionenaustauscherharze, Lithium enthaltende Metalloxide, hohle Glasmikrosphäre, Silizium-Solgel, Titan-Solgel und/oder gemischtes Titan als anorganische Stoffe,
- Kohlenstoff, aktivierte Holzkohle und/oder Diamantpuder als auf Kohlenstoff basierende Stoffe und/oder
- Polybutadien und/oder Polysiloxan als Elastomere.

18. Eine wirkstoffundurchlässige Deckschicht und/oder eine abziehbare Schutzschicht eines transdermalen therapeutischen Systems nach Anspruch 14 oder 15, **gekennzeichnet durch** einen Absorptionsmittelgehalt von 10 bis 70 Gew. % bezogen auf das Gesamtgewicht der Mischung aus Polymer, Kanalbildner und Absorptionsmittel.

19. Eine wirkstoffundurchlässige Deckschicht und/oder eine abziehbare Schutzschicht eines transdermalen therapeutischen Systems nach mindestens einem der Ansprüche 1 und 3 bis 18, **gekennzeichnet durch** eine Schichtdicke von 5 bis 100 µm, insbesondere 15 bis 40 µm, bevorzugt 15 µm.

20. Eine wirkstoffundurchlässige Deckschicht (und/oder eine abziehbare Schutzschicht) eines transdermalen therapeutischen Systems nach einem der Ansprüche 2 bis 19, **gekennzeichnet dadurch, daß** die für die wirkstoffundurchlässige Deckschicht verwendete Folie auf der Ober- und/oder Unterseite mit der Mischung beschichtet ist.

21. Eine (wirkstoffundurchlässige Deckschicht und/oder eine) abziehbare Schutzschicht eines transdermalen therapeutischen Systems nach einem der Ansprüche 2 bis 19, **gekennzeichnet dadurch, daß** die für die abziehbare Schutzschicht verwendete Folie auf der nach außen gerichteten Seite mit der Mischung beschichtet ist.

22. Transdermales therapeutisches System in Form eines Matrixsystems oder Membransystems, **gekennzeichnet durch** eine wirkstoffundurchlässige Deckschicht und/oder eine abziehbare Schutzschicht bestehend aus einer Mischung aus mindestens einem Polymer und eingebettetem Absorptionsmittel und Kanalbildner.

23. Transdermales therapeutisches System nach Anspruch 22, **gekennzeichnet durch** eine Deckschicht und/oder eine Schutzschicht gemäß einem der Ansprüche 1 oder 3 bis 21.

24. Transdermales therapeutisches System in Form eines Matrixsystems oder Membransystems, **gekennzeichnet durch** eine wirkstoffundurchlässige Deckschicht und/oder eine abziehbare Schutzschicht bestehend aus einer Folie, die mit einer Mischung (Polymerträger) aus mindestens einem Polymer, Kanalbildnern und Absorptionsmitteln vollflächig oder in Mustern beschichtet ist.

25. Transdermales therapeutisches System nach Anspruch 24, **gekennzeichnet durch** eine Deckschicht und/oder eine Schutzschicht gemäß einem der Ansprüche 2 bis 21.

26. Transdermales therapeutisches System nach einem der Ansprüche 22 bis 25, **dadurch gekennzeichnet, daß** es sich um ein Matrixsystem mit
- einer wirkstoffundurchlässigen Deckschicht,
- einer oder mehreren wirkstoffhaltigen selbstklebenden Matrixschicht(en) oder einer oder mehreren wirkstoffhaltigen Matrixschicht(en), die mit einem Haftkleber beschichtet sind, und
- einer abziehbaren Schutzschicht
handelt.

27. Transdermales therapeutisches System nach einem der Ansprüche 22 bis 25, **dadurch gekennzeichnet, daß** es sich um ein Membransystem mit
- einer undurchlässigen Deckschicht,
- einem wirkstoffhaltigen Reservoir oder einer wirkstoffhaltigen Reservoirschicht,
- einer mikroporösen oder semipermeablen Membran und
- einer fakultativen Haftklebeschicht
handelt.

28. Verfahren zur Herstellung eines transdermalen therapeutischen Systems mit wirkstoffundurchlässiger Deckschicht und abziehbarer Schutzschicht, bei dem man einen Polymerträger mit einem Gehalt an Absorptionsmittel und Kanalbildner vollflächig oder in Mustern auf die wirkstoffundurchlässige Deckschicht und/oder die abziehbare Schutzschicht aufbringt.

29. Verfahren nach Anspruch 28, bei dem man die Oberseite und/oder die Unterseite der wirkstoffundurchlässigen Deckschicht mit dem Polymerträger beschichtet.

30. Verfahren nach Anspruch 28 und/oder 29, bei dem man die Außenseite der abziehbaren Schutzschicht mit dem Polymerträger beschichtet.

31. Verfahren zur Herstellung eines transdermalen therapeutischen Systems mit wirkstoffundurchlässiger Deckschicht und abziehbarer Schutzschicht, bei dem man die wirkstoffundurchlässige Deckschicht und/oder die abziehbare Schutzschicht mit Absorptionsmittel und Kanalbildner versieht.

32. Verfahren nach einem der vorhergehenden Ansprüche, bei dem man als transdermales therapeutisches System ein Matrix- oder ein Membransystem herstellt.

33. Verfahren nach Anspruch 32, bei dem man ein transdermales therapeutisches System mit einer oder mit mehreren selbstklebenden Matrixschichten herstellt.

34. Verfahren nach mindestens einem der Ansprüche 28 bis 32, bei dem man ein transdermales therapeutisches System mit einer oder mehreren Matrixschichten herstellt, die mit einem Haftkleber beschichtet sind.

## Claims

1. A cover layer that is impermeable to the active substances and/or a removable protective layer of a transdermal therapeutic system, wherein the cover layer and/or protective layer is **characterized by** a mixture of at least one polymer and at least one embedded absorbing agent and at least one embedded channel-forming agent.

2. The cover layer that is impermeable to the active substances in the form of a film and/or a removable protective layer in the form of a film of a transdermal therapeutic system, **characterized in that** the film is coated with a mixture of at least one polymer, at least one channel-forming agent, and at least one absorbing agent, over the entire surface or in patterns.

3. The cover layer that is impermeable to the active substances and/or a removable protective layer in accordance with Claims 1 or 2, **characterized in that** a thermoplast or a crosslinkable or crosslinked polymer, respectively, is the polymer in the mixture of the polymer, absorbing agents, and channel-forming agents.

4. The cover layer that is impermeable to the active substances and/or a removable protective layer in accordance with Claims 2 or 3, **characterized in that** the cover layer and/or the protective layer have been coated with a mixture (polymer support) consisting of at least one thermoplast, at least one channel-forming agent, and at least one absorbing agent.

5. The cover layer that is impermeable to the active substances and/or a removable protective layer in accordance with Claims 2 or 3, **characterized in that** the cover layer and/or the protective layer have been coated, whether in a cold or hot state, with a mixture (polymer support) consisting of
(i) at least one thermally crosslinkable polymer, at least one channel-forming agent, and at least one absorbing agent, or
(ii) at least one polymer which can be crosslinked by means of radiation, in particular UV radiation, at least one channel-forming agent, and at least one absorbing agent, and
in either case, the crosslinking has been performed thereinafter.

6. The cover layer that is impermeable to the active substances and/or a removable protective layer of a transdermal therapeutic system in accordance with one of the preceding Claims, **characterized in that** the polymer is a polyolefin, polyisoprene, polybutadiene, polybutene, polysiloxane, polyamide, ethylene vinyl acetate copolymer, ethylene methacrylate copolymer, polystyrene, polyester, polyanhydride, polyacrylate nitrile, polyacryl nitrile, polysulfonate, polyesteramide, polyacrylate ester, propylene maleic anhydride, polyethylene maleic anhydride, polyethylene urethane, polyethylene ethyl vinyl alcohol, polyethylene nylon, and/or polyurethane.

7. The cover layer that is impermeable to the active substances and/or a removable protective layer in accordance with Claim 6, **characterized in that** the polymer is polyethylene and/or polypropylene.

8. The cover layer that is impermeable to the active substances and/or a removable protective layer of a transdermal therapeutic system in accordance with one of the preceding Claims, **characterized by** a polymer content of 10 to 90 percent by weight, based on the total weight of the mixture of the polymer, channel-forming agents, and absorbing agents.

9. The cover layer that is impermeable to the active substances and/or a removable protective layer of a transdermal therapeutic system in accordance with one of the preceding Claims, **characterized in that** the channel-forming agents are hydrophilic substances.

10. The cover layer that is impermeable to the active substances and/or a removable protective layer of a transdermal therapeutic system in accordance with Claim 9, **characterized in that** the channel-forming agents are polyglycols, ethyl vinyl alcohols, glycerin, pentaerythritol, polyvinyl alcohols, polyvinyl pyrrolidone, vinyl pyrrolidone, N-methyl pyrrolidone, polysaccharides, saccharides, and/or sugar alcohols.

11. The cover layer that is impermeable to the active substances and/or a removable protective layer in accordance with Claim 10, **characterized by**
- polyethylene glycol and/or polypropylene glycol as a polyglycol, and/or
- glucose, mannose, galactose and/or fructose as a saccharide, and/or
- mannitol, sorbitol, hexitol, dulcitol, xylitol, ribitol and/or erythrol as a sugar alcohol.

12. The cover layer that is impermeable to the active substances and/or a removable protective layer of a transdermal therapeutic system in accordance with Claim 9, 10, or 11, **characterized by** a content of the channel-forming agents of 10 to 40 percent by weight based on the total weight of the mixture of the polymer, channel-forming agents, and absorbing agents.

13. The cover layer that is impermeable to the active substances and/or a removable protective layer of a transdermal therapeutic system in accordance with one of the preceding Claims, **characterized in that** the absorbing agent is a substance for the absorption of humidity, oxygen or of substances which are released by a transdermal therapeutic system during storage, in particular of highly odorous substances, especially amines or polymer monomers.

14. The cover layer that is impermeable to the active substances and/or a removable protective layer of a transdermal therapeutic system in accordance with one of the preceding Claims, **characterized in that** the absorbing agents are drying agents, metals, alloys, metal-coated particles, inorganic substances, ion-exchange resins, substances on the basis of carbon, in particular on the basis of elemental carbon, elastomers, semi-metals, and/or ceramic materials.

15. The cover layer that is impermeable to the active substances and/or a removable protective layer of a transdermal therapeutic system in accordance with Claim 14, **characterized in that** the physical drying agents include molecular sieves, silicon gels, earths, synthetic polymers and/or starches, in particular molecular sieves.

16. The cover layer that is impermeable to the active substances and/or a removable protective layer in accordance with Claim 15, **characterized in that** montmorrillimite earth or montmorrillonite earth or polymers that are used in baby diapers or molecular sieves with a pore size from 3 to 15 Å or silicon dioxide gel with a pore size of approx. 24 Å are used, as drying agents.

17. The cover layer that is impermeable to the active substances and/or a removable protective layer in accordance with Claim 14, **characterized in that**
- nickel, copper, aluminum, silver and/or gold are used as a metal or alloy,
- silver-coated copper, silver-coated nickel, and/or silver-coated glass microspheres are used as metal-coated particles,
- barium titanium trioxide, strontium titanium trioxide, silicon dioxide, aluminum oxide, zinc oxide, titanium dioxide, manganese oxide, copper oxide, antimony oxide, molten or melted silicon, amorphous molten or amorphous melted silicon, ion-exchange resins, lithium-containing metal oxides, hollow glass microspheres, silicon sol-gel, titanium sol-gel, and/or mixed titanium are used as inorganic substances,
- carbon, activated charcoal and/or diamond powder are used as carbon-based substances, and/or
- polybutadiene and/or polysiloxanes are used as elastomers.

18. The cover layer that is impermeable to the active substances and/or a removable protective layer of a transdermal therapeutic system in accordance with Claims 14 or 15, **characterized by** a content of the absorbing agent from 10 to 70 percent by weight based on the total weight of the mixture of the polymer, channel-forming agents, and absorbing agents.

19. The cover layer that is impermeable to the active substances and/or a removable protective layer of a transdermal therapeutic system in accordance with at least one of Claims 1 and 3 through 18, **characterized by** a layer thickness of 5 to 100 µm, in particular 15 to 40 µm, preferably 15 µm.

20. The cover layer that is impermeable to the active substances (and/or a removable protective layer) of a transdermal therapeutic system in accordance with one of Claims 2 through 19, **characterized in that** the film used for the cover layer that is impermeable to the active substances is coated with the mixture on its top side and/or bottom side.

21. The (cover layer that is impermeable to the active substances and/or a) removable protective layer of a transdermal therapeutic system in accordance with one of Claims 2 through 19, **characterized in that** the film that is used for the removable protective layer is coated with the mixture on its outward-facing side.

22. A transdermal therapeutic system in the form of a matrix system or membrane system, **characterized by** a cover layer that is impermeable to the active substances and/or a removable protective layer consisting of a mixture of at least one polymer and an embedded absorbing agent and channel-forming agent.

23. The transdermal therapeutic system in accordance with Claim 22, **characterized by** a cover layer and/or a protective layer in accordance with one of Claims 1 or 3 through 21.

24. The transdermal therapeutic system in the form of a matrix system or membrane system, **characterized by** a cover layer that is impermeable to the active substances and/or a removable protective layer comprising a film that has been coated with a mixture (polymer support) of at least one polymer, channel-forming agents, and absorbing agents over its entire surface or in patterns.

25. The transdermal therapeutic system in accordance with Claim 24, **characterized by** a cover layer and/or a protective layer in accordance with one of Claims 2 through 21.

26. The transdermal therapeutic system in accordance with one of Claims 22 through 25, **characterized in that** the system is a matrix system with
- a cover layer that is impermeable to the active substances,
- one or more active substance-containing self-adhesive matrix layer(s) or one or more active substance-containing matrix layer(s) that have been coated with a pressure-sensitive adhesive, and
- a removable protective layer.

27. The transdermal therapeutic system in accordance with one of Claims 22 through 25, **characterized in that** such system is a membrane system with
- an impermeable cover layer,
- an active substance-containing reservoir or an active substance-containing reservoir layer,
- a microporous or semipermeable membrane, and
- an optional pressure-sensitive adhesive layer.

28. A method for the manufacture of a transdermal therapeutic system with a layer that is impermeable to the active substances and a removable protective layer, **characterized in that** a polymer support containing absorbing agents and channel-forming agents is applied, over the entire surface or in patterns, to the cover layer that is impermeable to the active substances and/or the removable protective layer.

29. The method in accordance with Claim 28, **characterized in that** the top side and/or the bottom side of the cover layer that is impermeable to the active substances is coated with the polymer support.

30. The method in accordance with Claims 28 and/or 29, **characterized in that** the outside of the removable protective layer is coated with the polymer support.

31. The method for the manufacture of a transdermal therapeutic system with a cover layer that is impermeable to the active substances and a removable protective layer, **characterized in that** absorbing agents and channel-forming agents are incorporated in the cover layer that is impermeable to the active substances and/or the removable protective layer.

32. The method in accordance with one of the preceding Claims, **characterized in that**, as a transdermal therapeutic system, a matrix or membrane system is produced.

33. The method in accordance with Claim 32, **characterized in that** a transdermal therapeutic system with one or more self-adhesive matrix layers is produced.

34. The method in accordance with at least one of Claims 28 through 32, **characterized in that** a transdermal therapeutic system with one or more matrix layers is produced which are coated with a pressure-sensitive adhesive.

## Revendications

1. Couche de recouvrement imperméable à l'agent actif et/ou couche de protection ôtable d'un système thérapeutique transdermique, qui est/sont caractérisée/s par un mélange d'au moins un polymère, au moins un absorbant noyé dedans et au moins un agent de formation de canaux, noyé dedans.

2. Couche de recouvrement imperméable à l'agent actif, sous la forme d'une feuille, et/ou couche de protection ôtable, sous la forme d'une feuille, d'un système thérapeutique transdermique, **caractérisée en ce que** la feuille est revêtue sur toute la surface ou suivant des dessins, d'un mélange d'au moins un polymère, au moins un agent de formation de canaux et au moins un absorbant.

3. Couche de recouvrement imperméable à l'agent actif et/ou couche de protection ôtable selon la revendication 1 ou 2, **caractérisée par** une matière thermoplastique ou un polymère réticulable ou réticulé, en tant que polymère du mélange de polymère, d'absorbant et d'agent de formation de canaux.

4. Couche de recouvrement imperméable à l'agent actif et/ou couche de protection ôtable selon la revendication 2 ou 3, **caractérisée en ce qu'**elle/s a/ont été revêtue/s d'un mélange (support polymère) constitué d'au moins une matière thermoplastique, au moins un agent de formation de canaux et au moins un absorbant.

5. Couche de recouvrement imperméable à l'agent actif et/ou couche de protection ôtable selon la revendication 2 ou 3, **caractérisée en ce que**:
(i) elle/s a/ont été revêtue/s à froid ou à chaud d'un mélange (support polymère) constitué d'au moins un polymère réticulable sous l'action de la chaleur, au moins un agent de formation de canaux et au moins un absorbant, ou bien
(ii) elle/s a/ont été revêtue/s à froid ou à chaud d'un mélange (support polymère) constitué d'au moins un polymère réticulable par irradiation, en particulier par des rayons UV, au moins un agent de formation de canaux et au moins un absorbant, et dans chaque cas la réticulation a été réalisée ensuite.

6. Couche de recouvrement imperméable à l'agent actif et/ou couche de protection ôtable d'un système thérapeutique transdermique selon l'une quelconque des revendications précédentes, **caractérisée en ce que** le polymère est une polyoléfine, un polyisoprène, un polybutadiène, un polybutène, un polysiloxane, un polyamide, un copolymère d'éthylène et d'acétate de vinyle, un copolymère d'éthylène et de méthacrylate, un polystyrène, un polyester, un polyanhydride, un polyacrylonitrile, un polysulfonate, un polyesteramide, un polyacrylate, un polypropylène-anhydride maléique, un polyéthylène-anhydride maléique, un polyéthylène-uréthane, un polyéthylène-éthylvinylcarbinol, un polyéthylène-nylon et/ou un polyuréthane.

7. Couche de recouvrement imperméable à l'agent actif et/ou couche de protection ôtable selon la revendication 6, **caractérisée par** du polyéthylène et/ou du polypropylène en tant que polymère.

8. Couche de recouvrement imperméable à l'agent actif et/ou couche de protection ôtable d'un système thérapeutique transdermique selon l'une quelconque des revendications précédentes, **caractérisée par** une teneur en polymère de 10 à 90 % en poids, cette teneur étant rapportée au poids total du mélange de polymère, d'agent de formation de canaux et d'absorbant.

9. Couche de recouvrement imperméable à l'agent actif et/ou couche de protection ôtable d'un système thérapeutique transdermique selon l'une quelconque des revendications précédentes, **caractérisée par** des produits hydrophiles en tant qu'agent de formation de canaux.

10. Couche de recouvrement imperméable à l'agent actif et/ou couche de protection ôtable d'un système thérapeutique transdermique selon la revendication 9, **caractérisée en ce que** l'agent de fomation de canaux est constitué de polyglycols, d'éthylvinylcarbinols, de glycérine, de pentaérythritol, d'alcools polyvinyliques, de polyvinylpyrrolidone, de vinylpyrrolidone, de N-méthylpyrrolidone, de polysaccharides, de saccharides et/ou d'alcools de sucres.

11. Couche de recouvrement imperméable à l'agent actif et/ou couche de protection ôtable selon la revendication 10, **caractérisée par** du polyéthylèneglycol et/ou du polypropylèneglycol en tant que polyglycol, et/ou
du glucose, du mannose, du galactose et/ou du fructose en tant que saccharide, et/ou
du mannitol, du sorbitol, de l'hexitol, du dulcitol, du xylitol, du ribitol et/ou de l'érythritol an tant qu'alcool de sucre.

12. Couche de recouvrement imperméable à l'agent actif et/ou couche de protection ôtable d'un système thérapeutique transdermique selon l'une quelconque des revendications 9 à 11, **caractérisée par** une teneur en agent de formation de canaux de 10 à 40 % en poids, cette teneur étant rapportée au poids total du mélange de polymère, d'agent de formation de canaux et d'absorbant.

13. Couche de recouvrement imperméable à l'agent actif et/ou couche de protection ôtable selon l'une quelconque des revendications précédentes, **caractérisée en ce que** l'absorbant est un absorbant pour l'absorption de l'humidité, de l'oxygène ou de produits qui sont libérés par un système thérapeutique transdermique lors du stockage, en particulier des produits très odorants, en particulier des amines ou des monomères de polymère.

14. Couche de recouvrement imperméable à l'agent actif et/ou couche de protection ôtable d'un système thérapeutique transdermique selon l'une quelconque des revendications précédentes, **caractérisée en ce que** l'absorbant est constitué de desséchants, de métaux, d'alliages, de particules recouvertes de métal, de produits minéraux, de résines échangeuses d'ions, de produits à base de carbone, en particulier de carbone élémentaire, d'élastomères, de métalloïdes et/ou de matières céramiques.

15. Couche de recouvrement imperméable à l'agent actif et/ou couche de protection ôtable d'un système thérapeutique transdermique selon la revendication 14, **caractérisée en ce que** les desséchants physiques comprennent des tamis moléculaires, des gels de silice, des terres, des polymères synthétiques et/ou des amidons, en particulier des tamis moléculaires.

16. Couche de recouvrement imperméable à l'agent actif et/ou couche de protection ôtable selon la revendication 15, **caractérisée en ce que** le desséchant est constitué de terre de montmorrillimite ou de terre de montmorrillonite, ou de polymères qui sont utilisés dans les couches des bébés, ou de tamis moléculaires ayant une taille de pore de 3 à 15 angstrôms, ou de gel de silice ayant une taille de pore d'environ 24 angströms.

17. Couche de recouvrement imperméable à l'agent actif et/ou couche de protection ôtable selon la revendication 14, **caractérisée par** :
du nickel, du cuivre, de l'aluminium, de l'argent et/ou de l'or en tant que métal ou alliage,
du cuivre revêtu d'argent, du nickel revêtu d'argent et/ou des microsphères de verre revêtues d'argent en tant que particules revêtues de métal,
du trioxyde de baryum et de titane, du trioxyde de strontium et de titane, du dioxyde de silicium, de l'oxyde d'aluminium, de l'oxyde de zinc, du dioxyde de titane, de l'oxyde de manganèse, de l'oxyde de cuivre, de l'oxyde d'antimoine, du silicium fondu, du silicium fondu amorphe, des résines échangeuses d'ions, des oxydes métalliques contenant du lithium, des microsphères de verre creuses, des gels de sol au silicium, des gels de sol au titane et/ou du titane en mélange en tant que produits minéraux,
du carbone, du charbon de bois activé et/ou de la poudre de diamant en tant que produits à base de carbone, et/ou
du polybutadiène et/ou du polysiloxane en tant qu'élastomères.

18. Couche de recouvrement imperméable à l'agent actif et/ou couche de protection ôtable d'un système thérapeutique transdermique selon la revendication 14 ou 15, **caractérisée par** une teneur en absorbant de 10 à 70 % en poids, cette teneur étant rapportée au poids total du mélange de polymère, d'agent de formation de canaux et d'absorbant.

19. Couche de recouvrement imperméable à l'agent actif et/ou couche de protection ôtable d'un système thérapeutique transdermique selon l'une quelconque des revendications 1 et 3 à 18, **caractérisée par** une épaisseur de couche de 5 à 100 µm, en particulier de 15 à 40 µm, de préférence de 15 µm.

20. Couche de recouvrement imperméable à l'agent actif et/ou couche de protection ôtable d'un système thérapeutique transdermique selon l'une quelconque des revendications 2 à 19, **caractérisée en ce que** la feuille utilisée pour la couche de recouvrement imperméable à l'agent actif est revêtue du mélange sur la face supérieure et/ou la face inférieure.

21. Couche de recouvrement imperméable à l'agent actif et/ou couche de protection ôtable d'un système thérapeutique transdermique selon l'une quelconque des revendications 2 à 19, **caractérisée en ce que** la feuille utilisée pour la couche de protection ôtable est revêtue du mélange sur le côté dirigé vers l'extérieur.

22. Système thérapeutique transdermique sous la forme d'un système à matrice ou d'un système à membrane, **caractérisé par** une couche de recouvrement imperméable à l'agent actif et/ou une couche de protection ôtable, qui est/sont constituée/s d'un mélange d'au moins un polymère et d'un absorbant et un agent de formation de canaux, noyés dedans.

23. Système thérapeutique transdermique selon la revendication 22, **caractérisé par** une couche de recouvrement et/ou une couche de protection selon l'une quelconque des revendications 1 et 3 à 21.

24. Système thérapeutique transdermique sous la forme d'un système à matrice ou d'un système à membrane, **caractérisé par** une couche de recouvrement imperméable à l'agent actif et/ou une couche de protection ôtable, qui est constituée d'une feuille qui est revêtue sur toute la surface ou suivant des dessins, d'un mélange (support polymère) d'au moins un polymère, d'agents de formation de canaux et d'absorbants.

25. Système thérapeutique transdermique selon la revendication 24, **caractérisé par** une couche de recouvrement et/ou une couche de protection selon l'une quelconque des revendications 2 à 21.

26. Système thérapeutique transdermique selon l'une quelconque des revendications 22 à 25, **caractérisé en ce qu'**il s'agit d'un système à matrice comportant :
une couche de recouvrement imperméable à l'agent actif,
une ou plusieurs couches de matrice autocollantes, contenant l'agent actif, ou une ou plusieurs couches de matrice contenant l'agent actif, qui sont revêtues d'une colle adhésive, et
une couche de protection ôtable.

27. Système thérapeutique transdermique selon l'une quelconque des revendications 22 à 25, **caractérisé en ce qu'**il s'agit d'un système à membrane comportant :
une couche de recouvrement imperméable,
un réservoir contenant l'agent actif ou une couche réservoir contenant l'agent actif,
une membrane microporeuse ou semi-perméable, et
une couche de colle adhésive facultative.

28. Procédé de préparation d'un système thérapeutique transdermique comportant une couche de recouvrement imperméable à l'agent actif et une couche de protection ôtable, dans lequel on applique un support polymère contenant un absorbant et un agent de formation de canaux, sur toute la surface ou suivant des dessins, sur la couche de recouvrement imperméable à l'agent actif et/ou la couche de protection ôtable.

29. Procédé selon la revendication 28, dans lequel on revêt avec le support polymère la face supérieure et/ou la face inférieure de la couche de recouvrement imperméable à l'agent actif.

30. Procédé selon la revendication 28 et/ou 29, dans lequel on revêt avec le support polymère la face externe de la couche de protection ôtable.

31. Procédé de préparation d'un système thérapeutique transdermique comportant une couche de recouvrement imperméable à l'agent actif et une couche de protection ôtable, dans lequel on garnit la couche de recouvrement imperméable à l'agent actif et/ou la couche de protection ôtable d'un absorbant et d'un agent de formation de canaux.

32. Procédé selon l'une quelconque des revendications précédentes, dans lequel on prépare comme système thérapeutique transdermique, un système à matrice ou à membrane.

33. Procédé selon la revendication 32, dans lequel on prépare un système thérapeutique transdermique comportant une ou plusieurs couches de matrice autocollantes.

34. Procédé selon l'une quelconque des revendications 28 à 32, dans lequel on prépare un système thérapeutique transdermique comportant une ou plusieurs couches de matrice, qui sont revêtues d'une colle adhésive.
